# FASCICULE DE BREVET EUROPEEN

(11) **EP 0 539 456 B1**
(45) Date de publication et mention de la délivrance du brevet: **30.03.1994**
(21) Numéro de dépôt: 91913274.6
(22) Date de dépôt: 16.07.1991
(51) Int. Cl.: G01N 21/85, G01N 33/34

(54) **DISPOSITIF DE DETECTION EN CONTINU DES IMPURETES DE CONTRASTE CONTENUES DANS UNE MATIERE FLUIDE EN DEPLACEMENT**
VORRICHTUNG ZUR KONTINUIERLICHEN BESTIMMUNG VON IN EINEM SICH BEWEGENDEN FLIESSFÄHIGEM MATERIAL ENTHALTENEN KONTRASTVERUNREINIGUNGEN
DEVICE FOR CONTINUOUSLY DETECTING CONTRAST IMPURITIES CONTAINED IN A MOVING FLUID MATERIAL

(30) Priorité: 17.07.1990 FR 9009349
(43) Date de publication de la demande: 05.05.1993
(73) Titulaire: CENTRE TECHNIQUE DE L'INDUSTRIE DES PAPIERS CARTONS ET CELLULOSE ., F-38100 Grenoble (FR)
(72) Inventeur: SABATER, Jacques, F-91190 Gif/Yvette (FR); BAUDUIN, Serge, F-38700 La Tronche (FR)
(74) Mandataire: Laurent, Michel
(86) Numéro de dépôt international: FR9100584
(87) Numéro de publication internationale: WO9201924

(56) Documents cités:
- EP-A- 0 226 430
- WO-A-81/03224
- WO-A-86/05525
- US-A- 4 075 462
- PATENT ABSTRACTS OF JAPAN vol. 9, No. 133 (P-362)(1856) 8 June 1985 & JP, A, 60 015 541

## Description

L'invention concerne un dispositif apte à détecter et mesurer en continu les impuretés de contraste contenues dans une matière fluide en déplacement. Par contraste, on entend au sens physique du terme, le rapport de la valeur absolue de la différence entre la luminance de l'impureté et la luminance du fond ou de la matière fluide sur la luminance du fond. De manière encore plus spécifique, l'invention se rapporte à la détection et à la mesure des impuretés de contraste contenues dans la pâte à papier, préalablement à sa projection sur la toile de base, dans le cadre de la fabrication du papier.

Bien que dans la suite de la description, et dans les revendications, l'invention sera décrite plus spécifiquement en liaison avec l'industrie papetière, elle ne saurait se restreindre à cette seule application.

L'un des problèmes fondamentaux des papetiers, dans le cadre de la fabrication ou la réalisation de papier, notamment en continu, concerne la possibilité de mesurer le taux d'impuretés contenues dans la pâte avant que celle-ci ne se soit projetée sur la toile de base de la feuille de papier. Par taux d'impuretés, on entend notamment la quantité et la taille des impuretés détectées. Par "impuretés", on désigne notamment les impuretés de contraste, dont l'origine peut provenir d'une part, des éléments entrant dans la constitution de la pâte elle-même, tels que les grains de sables, et d'autre part, des éléments étrangers présents dans les pâtes recyclées issues notamment de vieux papiers, tels que par exemple les traces de vernis, les traces d'encres.

Jusqu'à présent, afin de déterminer le taux d'impuretés contenues dans une pâte à papier, on avait l'habitude de prélever de la pâte à partir de laquelle on réalisait des formettes, c'est à dire des échantillons plans de pâte à papier, dans lesquels on comptait de visu, éventuellement à l'aide d'une loupe, les différentes impuretés présentes dans la pâte. De fait, cette procédure se révèlait particulièrement longue et fastidieuse, la durée du comptage étant en outre augmentée de la durée de réalisation des formettes. En outre et surtout il s'agissait d'un comptage en différé par rapport à la chaîne de fabrication du papier, ne pouvant donner lieu qu'à des valeurs statistiques plus ou moins éloignées de la réalité. En outre, compte tenu du faible nombre de mesures effectuées au regard de la quantité de pâte fabriquée, l'erreur statistique était loin d'être négligeable.

Dans le document WO-86/05 525, on a proposé un dispositif pour la détection des impuretés contenues dans la pâte à papier, dans laquelle cette détection s'effectue également sur des échantillons prélevés de pâte. Cette détection est effectuée ligne après ligne et ce, au moyen d'une barrette d'éléments photosensibles, telle qu'une barrette de photodiodes. Or, pour qu'une saisie ligne après ligne permette la caractérisation dimensionnelle des impuretés, il importe que la vitesse de défilement de la pâte à papier devant la barrette d'éléments photosensibles soit relativement lente afin de permettre la saisie de lignes jointives et de permettre de reconstituer ainsi une information représentative de l'aire de ces impuretés. Or, imprimer une vitesse lente aux déplacements de la pâte implique des conséquences incompatibles avec l'observation optique recherchée. En effet, cela provoque des phénomènes de sédimentation, de décantation et de floculation néfastes à l'observation. En outre, ces phénomènes altèrent les propriétés propres à la pâte. De la sorte, ce dispositif n'est pas intégrable directement sur une ligne de fabrication de papier.

On a également décrit dans le périodique spécialisé "PAPER and TIMBER" du mois de Mai 1989, pages 477 à 480, un dispositif d'analyse destiné à assurer la détection des impuretés dans la pâte à papier. Ce dispositif comprend fondamentalement une source lumineuse destinée à éclairer l'échantillon, notamment la pâte à papier sur une formette. La lumière transmise à travers ledit échantillon est captée par des optiques adaptés, et est analysée au moyen d'une caméra haute résolution, associée à un circuit à transfert de charges. Les signaux captés par la caméra sont alors numérisés, puis traités au moyen d'un système de traitement typiquement constitué d'un microordinateur associé à un logiciel adapté.

Si certes ce dispositif permet d'améliorer la qualité de la détection des impuretés, en revanche, il n'est toujours pas intégrable directement sur une chaîne de fabrication de papier. En effet, tel qu'il ressort de l'étude de ce document, il est encore nécessaire de prélever des échantillons et de réaliser des formettes, et même si ces prélèvements sont effectués de manière périodique et rapprochée, ils n'en constituent pas moins un obstacle à la réalisation d'une analyse en continu de la teneur en impuretés d'une pâte à papier. En outre, le nombre de formettes analysées est insuffisant pour assurer une niveau de précision convenable ; en effet, les résultats de ce comptage sont entachés d'une erreur statistique, qui est d'autant plus importante que le nombre compté est faible.

On a proposé dans le document WO-81/03224, un dispositif d'analyse optique d'un fluide en défilement, dans lequel les particules sont détectées au moyen d'un microscope associé à une caméra CCD, le microscope analysant la lumière transmise à travers le fluide. De fait, ce dispositif fonctionne en transmission, de sorte que certaines impuretés ne peuvent pas être détectées, notamment les flocs, et partant ne sont pas comptabilisées. En outre, s'il permet certes de détecter certaines particules, en revanche, il ne peut déterminer leur taille, et a fortiori leur surface apparente.

L'invention vise à pallier ces inconvénients. Elle propose un dispositif pour la détection et la mesure en continu des impuretés de contraste contenues dans une matière fluide en déplacement, et notamment dans la pâte à papier en défilement, qui puisse être intégré dans une installation à but déterminé, et notamment dans une chaine de fabrication de papier. Elle propose plus particulièrement un dispositif apte à mesurer la surface apparente de chaque impureté.

Ce dispositif, dans lequel la matière fluide traverse une canalisation, destinée à guider ladite matière d'un lieu à un autre, et munie sur l'une de ses faces d'une fenêtre transparente de dimensions déterminées, comprend :
- une source lumineuse impulsionnelle du type flash, destinée à éclairer ladite fenêtre transparente ;
- une caméra, dont le balayage trame est synchronisé avec la dite source lumineuse impulsionnelle, apte à détecter au niveau de la fenêtre éclairée, les impuretés de contraste ;
- des moyens de numérisation, associée à ladite caméra, et destinés à transformer les signaux analogiques issus de la caméra en signaux numériques ;
- un ensemble de traitement, dans la mémoire duquel les moyens de numérisation stockent les signaux numériques, et destiné à extraire de ces signaux la dimension et la quantité des impuretés de contraste présentes dans ladite matière au niveau de la fenêtre ;
- enfin, une sortie des signaux dudit ensemble de traitement pour la restitution de ces informations.

Ce dispositif se caractérise :
- en ce que la caméra est située en regard de la fenêtre du même coté que la source lumineuse impulsionnelle par rapport à ladite fenêtre, afin que la détection et la mesure des impuretés de contraste s'effectuent par rétrodiffusion, la surface apparente de chacune desdites impuretés étant extraite de cette mesure,
- et, en ce que la source lumineuse impulsionnelle est associé avec un élément intégrateur, destiné à assurer un éclairement uniforme de la totalité de la surface de la fenêtre de la canalisation, ledit élément intégrateur étant positionné au voisinage et au contact de ladite fenêtre.

En d'autres termes, l'invention consiste, par le biais d'éléments certes connus en soi, à maintenir constantes les conditions d'analyse et de détection des impuretés, tout en s'affranchissant de la réalisation d'échantillons. De la sorte, on obtient des résultats beaucoup plus précis et beaucoup plus représentatifs de la réalité, et ce en ligne sur l'installation. En outre, les conditions de mesure et de détection sont optimisées de par la réalisation de la détection en rétrodiffusion. En effet, une telle mesure effectuée en lumière rétrodiffusée permet de détecter les amas de fibres, généralement désignés sous l'expression en langue anglaise "flocs", contenus dans le fluide en déplacement, et notamment dans la pâte à papier, qui, dans le cadre d'une mesure effectuée en transmission, apparaissent sous la forme de masses sombres, risquant ainsi d'être considérés pour des impuretés, et donc, de fausser l'analyse finale.

Selon une forme avantageuse de l'invention, on munit le dispositif d'un fond diffusant, présentant une caractéristique de diffusion adaptée au fluide en déplacement, et notamment à la pâte à papier, ce qui réduit considérablement le contraste entre les flocs et le fond, et de ce fait, optimise la détection des impuretés.

Dans ce cas, la canalisation a une section rectangulaire, dont l'une des faces latérales reçoit ladite fenêtre, et dont la face latérale opposée comporte également une fenêtre transparente située en vis à vis de ladite fenêtre, et contre laquelle est appliquée extérieurement par rapport à la canalisation,un tel fond de caractéristique de diffusion adaptée à la matière fluide en défilement.

Avantageusement, en pratique :
- la synchronisation de l'éclairement de la source lumineuse impulsionnelle avec la caméra est effectuée au niveau de l'ensemble de traitement ;
- l'élément intégrateur est constitué d'une sphère, percée de trois orifices, permettant respectivement le passage de la source lumineuse impulsionnelle, le passage de la caméra et enfin définissant l'ouverture correspondant à la fenêtre de la canalisation, ladite caméra étant située diamétralement opposée à la fenêtre de la canalisation, la sphère tangentant ledit tube au niveau de cette fenêtre ;
- La sortie de l'ensemble de traitement est reliée à un organe de restitution sensorielle, destiné à permettre la visualisation des informations extraites de la caméra par ledit ensemble ;
- l'ensemble de traitement est constitué par un micro-ordinateur.

La manière dont l'invention peut être réalisée et les avantages qui en découlent, ressortiront mieux de l'exemple de réalisation qui suit à l'appui des figures annexées dans lesquelles :
- la figure 1 est une vue schématique du mode de fonctionnement du dispositif conforme à l'invention ;
- la figure 2 est une représentation graphique d'un exemple de mesure d'impuretés conforme à l'invention ;
- la figure 3 est une vue schématique en coupe transversale de la sphère intégratrice selon l'invention.

La description qui suit est plus particulièrement orientée vers un dispositif pour la détection des impuretés de contraste contenues dans la pâte à papier. Toutefois, l'invention ne saurait à se limiter à cette seule application.

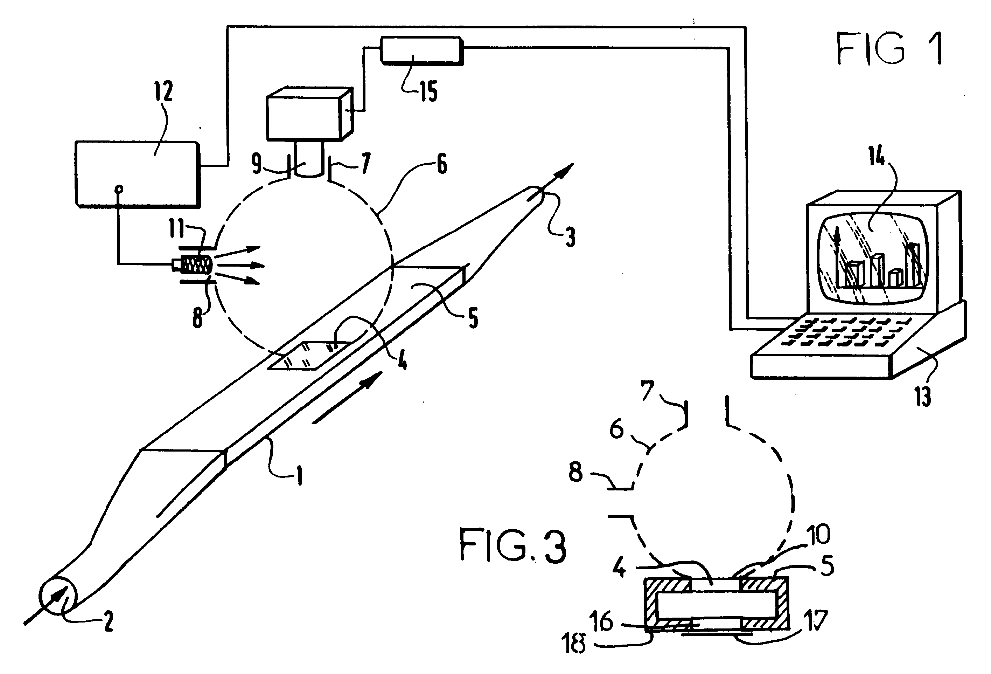

Selon l'invention, et comme on peut le voir sur la figure 1, le dispositif comprend tout d'abord une canalisation (1) de dérivation sur la conduite principale, destinée à acheminer la pâte de son lieu de fabrication vers le lieu de mesure. Dans une autre forme de réalisation, on peut envisager d'effectuer la mesure directement sur la conduite principale.

Cette canalisation présente au niveau du dispositif conforme à l'invention une section transversale rectangulaire, dont l'épaisseur réglable en fonction de la concentration de la pâte est typiquement de l'ordre de cinq millimètres. Ainsi, la pâte à papier arrive dans le tube (1) par son ouverture proximale (2) et sort de celui-ci par son ouverture distale (3) à une vitesse suffisante pour éviter tout risque de floculation. Typiquement, cette vitesse est de l'ordre de 2 à 5 m.s⁻¹.

Elle présente au niveau de sa face supérieure (5) une ouverture (4) de forme rectangulaire ou carrée de quatre à cinq centimètres de côté, close par un matériau transparent à la lumière, par exemple en verre.

Symétriquement, et comme on peut le voir sur la figure 3, la face opposée (18) à la face (5) comporte également une fenêtre (16) réalisée en un matériau transparent, en verre par exemple, et est recouverte à l'extérieur d'une feuille (17) de papier ou de tout autre matériau de couleur la plus proche possible de celle de la pâte à papier, afin de générer un coefficient de rétrodiffusion très proche de celui de la pâte et ce, pour s'affranchir lors de la détection des signaux correspondants aux grains constitutifs de la pâte.

Selon l'invention, le dispositif comprend également une sphère intégratrice (6) qui vient tangenter le tube (1) au niveau de la fenêtre (4), et qui est fixée par tout moyen approprié audit tube (1). Cette sphère intégratrice (6), réalisée en un métal quelconque, est revêtue à l'intérieur d'un matériau blanc et diffusant, afin de permettre un éclairage uniforme du système et notamment de la fenêtre (4), quel que soit la position de la source lumineuse, décrite ultérieurement, au sein de cette sphère.

En outre, cette sphère est percée de trois orifices (7) et (8), dont l'un (7) est diamétralement opposé à la fenêtre (4). Cet orifice (7) est destiné à recevoir l'objectif d'une caméra (9), associée à un circuit à transfert de charge.

L'autre orifice (8) est destiné à recevoir un flash (11) jouant le rôle de source lumineuse, ledit flash étant alimenté de manière conventionnelle par une alimentation (12). Avantageusement, on choisit un flash tel, que la durée des éclairs qu'il émet soit suffisamment bref pour que l'erreur de bougé soit inférieure à la résolution de la caméra. En effet, comme il sera décrit plus en détail ultérieurement, on vise par ce biais à minimiser les erreurs de bougé, en d'autres termes, les variations de détection au niveau de la caméra, liées au défilement de la pâte. De fait, ces erreurs doivent être inférieures à la résolution de l'image pour obtenir des valeurs de détection et de mesure précises et cohérentes.

Le dernier orifice (10) de forme rectangulaire, correspqnd à la fenêtre (4).

La caméra (9), est reliée à un système de traitement (13), constitué par exemple par un microordinateur. Ce microordinateur est géré par un logiciel adapté. De façon plus spécifique, les signaux lumineux captés par la caméra (9) subissent un premier traitement, à savoir leur transformation en signaux analogiques. Ces signaux analogiques, sont alors transformés en signaux numériques au moyen d'un carte de numérisation (15) associée à la caméra. Ce sont ces signaux numériques qui sont traités par le microordinateur (13).

Avantageusement, le flash (11) et la caméra (9) sont activés en synchronisme et ce, sous contrôle dudit microordinateur (13). Plus précisément, l'éclairement du flash est synchronisé avec le balayage trame de la caméra, autrement dit, avec le signal analogique ou numérique qui donne le départ au balayage ligne à ligne de la caméra pour chaque image successive. Ainsi, à chacun des éclairs du flash (11) qui, compte tenu de la présence de la sphère intégratrice (6) éclaire de manière uniforme la fenêtre (4) en dessous de laquelle défile la pâte à vitesse constante, la caméra (9) capte les informations lumineuses rétrodiffusées par la pâte. Ces informations concernant essentiellement les impuretés de contraste contenues dans la pâte au niveau de la fenêtre (4), numérisées par la carte de numérisation (15), sont alors stockées par cette dernière dans la mémoire du microordinateur. En d'autres termes, le microordinateur reçoit dans sa mémoire une carte graphique numérisée, représentative de la vision de la caméra (9) à travers la fenêtre (4). Une résolution typique du dispositif ainsi constitué est voisine cent micromètres (100 µm), pour une carte de numérisation de 512 x 512 pixels.

Cette cartographie numérisée transmise et stockée dans la mémoire du microordinateur (13), est traitée par ce dernier afin de détecter la présence des impuretés de contraste ainsi que leur taille, et surtout leur surface apparente. Comme déjà dit, ce traitement est géré par un logiciel, d'un type en soi connu pour cette application.

Une fois cette analyse réalisée, les résultats peuvent être affichés sur l'écran de visualisation (14), voire imprimés au moyen d'une imprimante traditionnelle. Ces résultats peuvent également transiter par une sortie sous forme de signaux analogiques ou numériques, pour être exploités par tout système de traitement.

Comme déjà dit, la caméra et le flash sont synchronisés et activés par le microordinateur. Avantageusement, lorsque le traitement d'une image est terminé, le microordinateur ordonne à l'ensemble : caméra/flash de lui restituer une nouvelle image numérisée. De la sorte, les intervalles de temps séparant deux analyses successives sont réduits, en vue d'une optimisation de la précision du dispositif par diminution de l'erreur statistique dûe à l'augmentation du nombre de mesures effectuées.

Avantageusement, le dispositif comprend un deuxième écran, associé à la caméra (9), afin de parfaire la mise au point de l'image lors de l'installation du dispositif.

On a précisé dans le cadre de l'exemple précédent que l'épaisseur du tube parallélépipédique est de l'ordre de cinq millimètres. Toutefois, cette épaisseur peut être adaptée en fonction d'une part, de l'opacité de la pâte, et d'autre part et surtout de la profondeur de champ de la caméra. Ainsi, lorsque l'on désire observer des impuretés de très petites dimensions, il est tout à fait possible d'adapter à la caméra un objectif type "objectif de microscope". Dans ce cas, il devient nécessaire de réduire l'épaisseur du tube, par exemple à une valeur voisine de 0,5 millimètre.

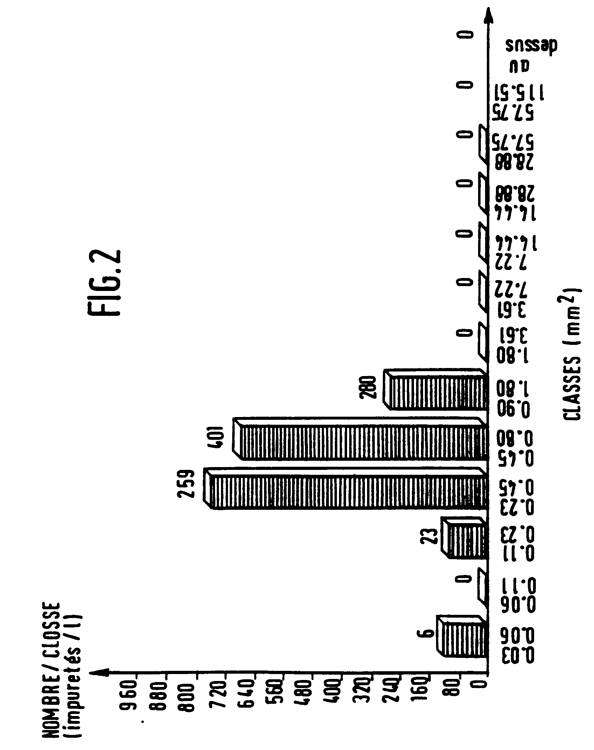

On a représenté dans la figure 2 un exemple de restitution classique d'une analyse de la teneur en impuretés d'une pâte à papier, et de la dimension de ces impuretés. Cette analyse se présente sous la forme d'un histogramme. On a représenté en abscisse différentes classes de taille de particules, en millimètres carré (mm²), et en ordonnée, le nombre d'impuretés dans chacune des classes par litre de pâte. En outre, on a représenté pour chacune des classes le nombre total d'impuretés.

Ainsi, le dispositif conforme à l'invention offre des possibilités appréciables dans la mesure où il permet de connaître en permanence dans le cadre d'une chaine de fabrication de papier, la teneur en impuretés ainsi que leur taille, ou leur surface apparente. De fait, l'utilisation du flash permet de figer l'image et ainsi d'obtenir des mesures plus précises. Compte tenu de la capacité des flash existants, on peut tout à fait envisager de faire défiler la pâte rapidement.

Ce dispositif permet ainsi d'effectuer ces différentes mesures en temps réel, sans délai de réalisation d'échantillons, ce que ne permettaient pas les dispositifs connus jusqu'alors. En outre, ce dispositif est adaptable soit directement sur la canalisation principale de la matière en défilement, soit sur une ligne de piquage montée en parallèle avec celle-ci, et ce au gré des utilisateurs, en fonction des impératifs d'encombrement et de résultats recherchés.

## Revendications

1. Dispositif pour la détection et la mesure en continu des impuretés de contraste contenues dans une matière fluide en déplacement, dans lequel la matière fluide traverse une canalisation (1) muni sur l'une de ses faces (5) d'une fenêtre transparente (4) de dimensions déterminées, et comprenant :
- une source lumineuse impulsionnelle (11) du type flash, destinée à éclairer ladite fenêtre (4) ;
- une caméra (9), dont le balayage trame est synchronisé avec la source lumineuse impulsionnelle (11), apte à détecter au niveau de la fenêtre (4) éclairée, les impuretés de contraste ;
- des moyens de numérisation (15), associés à la caméra (9), destinés à transformer les signaux analogiques issus de celle-ci en signaux numériques ;
- un ensemble de traitement (13), relié à ladite caméra (9), et destiné à extraire des signaux numériques stockés dans sa mémoire par lesdits moyens de numérisation (15), la dimension et la quantité des impuretés de contraste présentes dans la matière fluide au niveau de ladite fenêtre (4) ;
- enfin, une sortie ménagée au niveau dudit ensemble de traitement pour la restitution de ces informations sous forme visuelle, analogique ou numérique,
carcatérisé :
- en ce que la caméra (9) est située en regard de la fenêtre (4) du même coté que la source lumineuse impulsionnelle (11) par rapport à la fenêtre (4), afin que la détection et la mesure des impuretés de contraste s'effectuent par rétrodiffusion, la surface apparente de chacune desdites impuretés étant extraite de cette mesure,
- et, en ce que la source lumineuse impulsionnelle (11) est associé avec un élément intégrateur (6), destiné à assurer un éclairement uniforme de la totalité de la surface de la fenêtre (4) de la canalisation (1), ledit élément intégrateur étant positionné au voisinage et au contact de ladite fenêtre (4).

2. Dispositif selon la revendication 1, caractérisé en ce que la synchronisation de l'éclairement du flash (11) avec la caméra (9) est effectuée au niveau de l'ensemble de traitement (13).

3. Dispositif selon l'une des revendications 1 et 2, caractérisé en ce que en ce que l'élément intégrateur (6) est une sphère percée de trois orifices (7,8,10), dont l'un (7) est diamétralement opposé à la fenêtre (4), les orifices (7,8) étant destinés à permettre le passage respectif de la caméra (9) et du flash (11), l'orifice (10) définissant une ouverture correspondant à la fenêtre (4), ladite sphère tangentant la canalisation (1) au niveau de la fenêtre (4).

4. Dispositif selon l'une des revendications 1 à 3, caractérisé en ce que la canalisation (1) a une section rectangulaire, dont l'une des faces latérales (5) reçoit la fenêtre (4), et dont la face latérale opposée (18) comporte également une fenêtre transparente (16) située en vis à vis de la fenêtre (4), et contre laquelle est appliquée extérieurement par rapport à la canalisation (1), un fond diffusant (17) de caractéristique de diffusion adaptée à la matière fluide en défilement .

5. Dispositif selon l'une des revendications 1 à 4, caractérisé en ce que la sortie de l'ensemble de traitement (13) est reliée à un organe de restitution sensorielle (14), destiné à permettre la visualisation des informations extraites de la caméra (9) par ledit ensemble (13).

6. Dispositif selon l'une des revendications 1 à 5, caractérisé en ce que l'ensemble de traitement (13) est constitué par un microordinateur.

7. Dispositif selon l'une des revendications 1 à 6, caractérisé en ce qu'il comporte en outre un second organe de restitution sensorielle et notamment un écran, destiné à permettre, lors de l'installation du dispositif, la réalisation de la mise au point de la caméra (9) par rapport à la fenêtre (4) située en regard de celle-ci.

8. Dispositif selon l'une des revendications 1 à 7, caractérisé en ce qu'il est destiné à être intégré dans une chaîne de fabrication de papier, la matière fluide en défilement étant constitué par la pâte à papier.

## Patentansprüche

1. Vorrichtung zum kontinuierlichen Detektieren und Messen von Kontrastverunreinigungen, die in einem sich vorbeibewegenden flüssigen Medium enthalten sind, bei der das flüssige Medium eine Leitung (1) durchquert, die an einer ihrer Flächen (5) mit einem transparenten Fenster (4) von festgelegten Abmaßen versehen ist, mit:
- einer gepulsten Beleuchtungsquelle (11) nach Art eines Blitzgerätes, die dazu vorgesehen ist, das Fenster (4) zu beleuchten,
- einer Kamera (9), deren Teilbildabtastung mit der gepulsten Beleuchtungsquelle (11) synchronisiert ist und die dazu geeignet ist, auf der Höhe des beleuchteten Fensters (4) die Kontrastverunreinigungen zu detektieren,
- Digitalisiermitteln (15), die der Kamera (9) zugeordnet und dazu vorgesehen sind, die von dieser abgeleiteten analogen Signale in numerische Signale zu transformieren,
- einer Verarbeitungseinrichtung (13), die mit der Kamera (9) verbunden und dazu vorgesehen ist, aus den in ihrem Speicher von den Digitalisiermitteln (15) abgelegten numerischen Signalen die Größe und die Menge der Kontrastverunreinigungen zu extrahieren, die auf der Höhe des Fensters (4) in dem flüssigen Medium vorhanden sind,
- und schließlich einem im Bereich der Verarbeitungsvorrichtung vorgesehenen Ausgang für die Wiedergabe dieser Informationen in visueller, analoger oder numerischer Form,
dadurch gekennzeichnet,
- daß die Kamera (9) dem Fenster (4) gegenüberstehend bezogen auf das Fenster (4) auf der selben Seite wie die gepulste Beleuchtungsquelle (11) gelegen ist, so daß das Detektieren und das Messen der Kontrastverunreinigungen über Rückstreuung erfolgt, wobei die scheinbare Oberfläche einer jeden der genannten Verunreinigungen aus dieser Messung extrahiert wird,
- und dadurch, daß die gepulste Beleuchtungsquelle (11) mit einem Integratorelement (6) verbunden ist, das dazu vorgesehen ist, eine gleichmäßige Beleuchtung der Gesamtheit der Fläche des Fensters (4) der Leitung (1) sicherzustellen, wobei das Integratorelement in der Nachbarschaft des Fensters und in Kontakt mit dem genannten Fenster (4) angeordnet ist.

2. Vorrichtung nach Anspruch 1, dadurch gekennzeichnet, daß die Synchronisation der Beleuchtungsstärke des Blitzgerätes (11) mit der Kamera (9) im Bereich der Verarbeitungseinrichtung (13) bewirkt wird.

3. Vorrichtung nach einem der Ansprüche 1 oder 2, dadurch gekennzeichnet, daß das Integratorelement (6) eine von drei Öffnungen (7, 8, 10) durchbohrte Sphäre ist, von denen eine Öffnung (7) dem Fenster (4) diametral gegenüber gelegen ist, wobei die Öffnungen (7, 8) dazu vorgesehen sind, den Durchlaß der Kamera (9) bzw. des Blitzgerätes (11) zu ermöglichen, die Öffnung (10) eine dem Fenster (4) entsprechende Öffnung definiert und die Sphäre die Leitung (1) auf der Höhe des Fensters (4) berührt.

4. Vorrichtung nach einem der Ansprüche 1 bis 3, dadurch gekennzeichnet, daß die Leitung (1) einen rechtwinkligen Abschnitt umfaßt, dessen eine seitliche Fläche (5) das Fenster (4) aufnimmt und dessen gegenüberliegende seitliche Fläche (18) gleichfalls ein transparentes Fenster (16) umfaßt, das dem Fenster (4) gegenübergelegen ist und an dem bezogen auf die Leitung (1) von außen ein Streuboden (17) mit einer Streucharakteristik befestigt ist, die an das sich vorbeibewegende flüssige Medium angepaßt ist.

5. Vorrichtung nach einem der Ansprüche 1 bis 4, dadurch gekennzeichnet, daß der Ausgang der Verarbeitungseinrichtung (13) mit einem sensorischen Wiedergabeorgan verbunden ist, das dazu vorgesehen ist, die Darstellung der durch die Einrichtung (13) von der Kamera (9) extrahierten Informationen zu ermöglichen.

6. Vorrichtung nach einem der Ansprüche 1 bis 5, dadurch gekennzeichnet, daß die Verarbeitungseinrichtung (13) durch einen Mikrocomputer gebildet ist.

7. Vorrichtung nach einem der Ansprüche 1 bis 6, dadurch gekennzeichnet, daß sie unter anderem ein zweites sensorisches Wiedergabeorgan umfaßt, und zwar einen Bildschirm, der dazu bestimmt ist, während der Installation der Vorrichtung die Durchführung des Scharfeinstellens der Kamera (9) bezogen auf das Fenster (4) zu erlauben, das dieser gegenüberstehend angeordnet ist.

8. Vorrichtung nach einem der Ansprüche 1 bis 7, dadurch gekennzeichnet, daß sie dazu vorgesehen ist, in eine Straße zur Papierfertigung integriert zu werden, wobei das sich vorbeibewegende flüssige Medium durch den Papierstoffbrei gebildet ist.

## Claims

1. Device for continuously detecting contrast impurities contained in a moving fluid material, in which the fluid material passes through a duct (1) equipped on one of its faces (5) with a transparent window (4) of specific dimensions, and comprising:
- a pulsed slight-source (11) of the flash type, intended to illuminate said window (4);
- a camera (9) the field of sweep of which is synchronised with the pulsed light-source (11), capable of detecting the contrast impurities at the level of the illuminated window (4);
- digitalisation means (15) associated with the camera (9), intended to convert the analogue signals originating from the latter into digital signals;
- a processing unit (13) connected to said camera (9), and intended to extract from the digital signals, stored in its memory by the said digitalisation means (15), the dimensions and quantity of the contrast impurities present in the fluid material at the level of said window (4);
- finally, an output positioned at the level of said processing unit for reconverting this information into an analogue or digital visual form;
characterised in that the camera (9) is positioned facing the window (4) on the same side as the pulsed light-source (11) with respect to the window (4), so that detection and measurement of contrast impurities are effected by back-scatter, the apparent surface area of each of said impurities being extracted by this measurement;
- and in that the pulsed light-source (11) is associated with an integrating unit (6) intended to ensure uniform illumination of the entire surface of the window (4) of the duct (1), said integrating unit being positioned in the vicinity of and in contact with said window (4).

2. Device according to claim 1, characterised in that synchronisation of the illumination of the flash (11) with the camera (9) is effected at the level of the processing unit (13).

3. Device according to one of claims 1 and 2, characterised in that the integrating unit (6) is a sphere with three openings (7, 8, 10), one of which (7) is diametrally opposite the window (4), the openings (7, 8) being intended to allow passage respectively to the camera (9) and to the flash (11), the opening (10) defining an opening corresponding to the window (4), said sphere being tangential to the duct (1) at the level of the window (4).

4. Device according to one of claims 1 to 3, characterised in that the duct (1) is of rectangular cross-section, one of its lateral faces (5) receiving the window (4), and the opposite lateral face (18) of which likewise comprises a transparent window (16) situated facing the window (4), and against which there is applied externally with respect to the duct (1) a diffusing base (17) with a diffusion characteristic adapted to the moving fluid material.

5. Device according to one of claims 1 to 4, characterised in that the output of the processing unit (13) is connected to a sensory reconversion unit (14) intended to allow visualisation of the information extracted from the camera (9) by said unit (13).

6. Device according to one of claims 1 to 5, characterised in that the processing unit (13) is comprised by a microcomputer.

7. Device according to one of claims 1 to 6, characterised in that it comprises in addition a second sensory reconversion unit and in particular a screen intended, during installation of the device, to enable setting up of the camera (9) with respect to the window (4) situated facing the latter.

8. Device according to one of claims 1 to 7, characterised in that it is intended to be integrated into a paper production line, the moving fluid material being constituted by paper pulp.
